# EUROPÄISCHE PATENTSCHRIFT

(11) **EP 1 896 880 B1**
(45) Veröffentlichungstag und Bekanntmachung des Hinweises auf die Patenterteilung: **09.06.2010**
(21) Anmeldenummer: 06762142.5
(22) Anmeldetag: 22.06.2006
(51) Int. Cl.: G02B 3/14, A61B 3/09

(54) **KUNSTAUGE**
ARTIFICIAL EYE
OEIL ARTIFICIEL

(30) Priorität: 29.06.2005 EP 05014074
(43) Veröffentlichungstag der Anmeldung: 12.03.2008
(73) Patentinhaber: WaveLight AG, 91058 Erlangen (DE)
(72) Erfinder: GÖTZINGER, Volker, 90562 Heroldsberg (DE); SCHNALKE, Andreas, 90491 Nürnberg (DE); MICHLING, Arthur, 90766 Fürth (DE); HEIBERGER, Kurt, 90455 Nürnberg (DE)
(74) Vertreter: von Hellfeld, Axel
(86) Internationale Anmeldenummer: PCT/EP2006/006028
(87) Internationale Veröffentlichungsnummer: WO 2007/000280

(56) Entgegenhaltungen:
- WO-A-2004/049927
- WO-A1-2004/088613
- DE-A1- 19 623 270
- US-A1- 2002 196 558
- US-A1- 2004 227 063
- US-A1- 2005 002 113
- US-B1- 6 369 954
- PATENT ABSTRACTS OF JAPAN Bd. 013, Nr. 391 (P-926), 30. August 1989 (1989-08-30) -& JP 01 140118 A (MITSUBISHI HEAVY IND LTD), 1. Juni 1989 (1989-06-01)

## Beschreibung

Die Erfindung betrifft ein Kunstauge.

Der Stand der Technik (Produkte der Firmen Varioptic und Philips; US 6,369,954 und EP 1 019 758) kennt steuerbare Flüssigkeitslinsen, mit denen die Brechkraft im Bereich von etwa -15 dpt bis 30 dpt innerhalb von Zeitintervallen von wenigen Millisekunden mit Spannungen von bis zu 100 V steuerbar sind.

Die JP 011 40 118 beschreibt eine Linse, deren Oberfläche durch eine elastische Wand gebildet wird, die durch Änderung von elektrischen Spannungen deformierbar ist.

Die WO 2004/049927 beschreibt eine Vorrichtung zur Augenuntersuchung, die eine Flüssigkeitslinse enthält.

Die WO 2004/088613 und die US 2002/0085172 beschreiben Kunstaugen.

Der Erfindung liegt die Aufgabe zugrunde, ein Kunstauge bereitzustellen, mit dem umfangreiche und aufschlussreiche Messungen durchführbar sind zum Zwecke des Vergleichs mit Messungen an Augen *in vivo*.

Hierzu stellt die Erfindung ein Kunstauge bereit mit den Merkmalen des Patentanspruchs 1. Eine vorteilhafte Ausgestaltung des Kunstauges ist in dem vom Anspruch 1 abhängigen Anspruch 2 beschrieben.

Gemäß einer weiteren Ausgestaltung ist das Kunstauge zur Verbesserung der Messergebnisse und um insbesondere unter wechselnden Bedingungen reproduzierbare Messergebnisse zu erhalten, mit einer Laserstrahlquelle ausgerüstet zur Erzeugung eines Bündels aus einer Vielzahl paralleler Lichtstrahlen, einer CCD-Kamera zur Aufnahme von von den Lichtstrahlen nach Durchgang durch die Linsen erzeugten Bildern, und mit einem Rechner zur Verarbeitung der Bilder und zum Steuern des Flüssigkeitslinsensystems in Abhängigkeit von der Bildverarbeitung.

Nachfolgend werden Ausführungsbeispiele anhand der Zeichnung näher beschrieben. Es zeigt:
- Figur 1: schematisch einen Schnitt durch ein Flüssigkeitslinsensystem;
- Figur 2: schematisch eine Draufsicht auf die Elektrodenanordnung eines Flüssigkeitslinsensystems gemäß Figur 1;
- Figur 3: ein Kunstauge;
- Figur 4: eine messtechnische Ausrüstung eines Kunstauges gemäß Figur 3;
- Figur 5: ein Akkommodationsmessgerät; und
- Figur 6: ein Dioptriefernrohr.

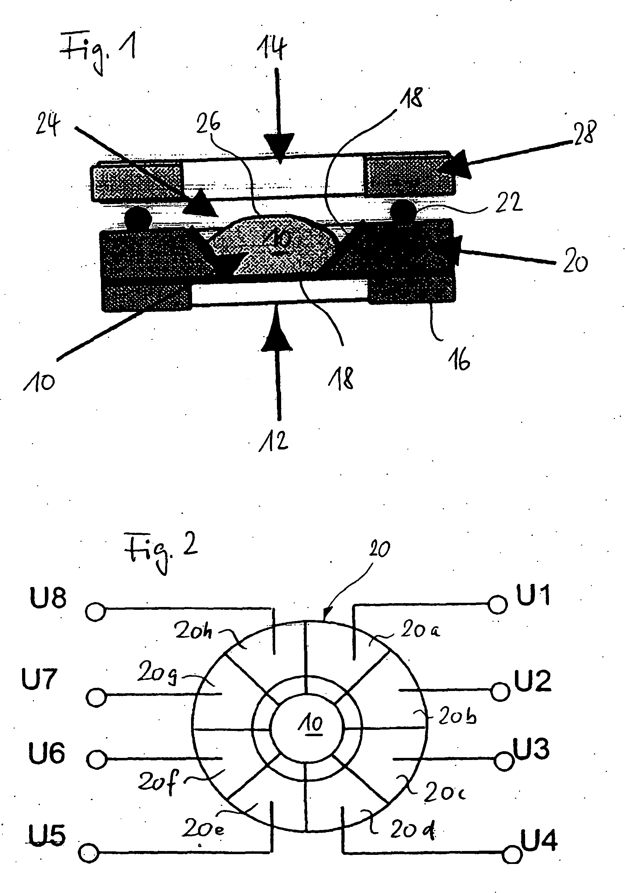

Gemäß Figur 1 ist ein Flüssigkeitstropfen 10, beim dargestellten Ausführungsbeispiel ein Öltropfen, zwischen zwei strahlungsdurchlässigen Fenstern 12, 14 angeordnet. Abzubildende Strahlung (Licht) wird durch den Flüssigkeitstropfen 10 in einstellbarer Weise gebrochen.

Der Flüssigkeitstropfen 10 ruht auf einer transparenten Zwischenlage 18, die wiederum von einem Stützring 16 abgestützt ist.

Um den Flüssigkeitstropfen 10 herum ist ein Elektrodenring 20 angeordnet, der in Figur 2 in Draufsicht dargestellt ist. Wie Figur 2 zeigt, besteht der Elektrodenring 20 beim dargestellten Ausführungsbeispiel aus acht Segmenten (20a, 20b, 20c, 20d, 20e, 20f, 20g, 20h). Die genannten Segmente bilden einen geschlossenen Kreis. Figur 2 zeigt auch die jeweils an die einzelnen Segmenten selektiv anlegbaren elektrischen Spannungen U1, U2, U3, U4, U5, U6, U7 und U8. Durch Anlegung unterschiedlicher Spannungen an die einzelnen Segmente lässt sich die Oberfläche 26 des Flüssigkeitstropfens 10 ändern, sodass die Brecheigenschaften des Flüssigkeitstropfens 10 wahlweise einstellbar sind. Ein Ring 28 bildet eine, z.B. an "Masse" liegende, Gegenelektrode für die einzelnen Elektroden 20a, ..., 20h. Um zum Beispiel eine Zylinderlinse nachzubilden, müssen die Spannungen U2, U3, U6, U7 auf ein erstes Spannungspotential gelegt werden und die Spannungen U1, U4, U5 und U8 der anderen Segmente auf ein anderes Spannungspotential. Auf diese Weise lässt sich mit einem Flüssigkeitslinsensystem gemäß den Figuren 1 und 2 ein Linsenfehler elektrische einstellen, zum Beispiel eine Sphäre, ein Zylinder, Koma, oder auch weitere Fehler höherer Ordnung. Je mehr Segmente analog Figur 2 um den Flüssigkeitstropfen 10 herum angeordnet sind, umso höher ist die örtliche Auflösung der einzustellenden Linsenfehler. Die Anordnung der einzelnen Elektrodensegmente (20a, ..., 20h) muss nicht notwendig rotationssymmetrisch sein. Das Ausführungsbeispiel gemäß Figur 2 zeigt zwar eine acht-zählige Rotationssymmetrie der Elektroden, derart, dass jeweils zwei Elektroden diametral in Bezug auf den Flüssigkeitstropfen 10 liegen. In Abwandlung dieses Ausführungsbeispiels kann, insbesondere zur Erzeugung von asphärischen Verformungen des Flüssigkeitstropfens 10, vorgesehen sein, die Elektroden, abweichend von der Darstellung gemäß Figur 2 so anzuordnen, dass zur Korrektur von typischen Abbildungsfehlern des Auges in Bezug auf die optische Achse asymetrische Verformungen und auch lokal in Abhängigkeit vom Sehfehler des Patienten gezielt Verformungen erreicht werden. Es wird also eine gezielte Verformung der Grenzfläche 26 der Flüssigkeitslinse 10 erzielt.

Nachfolgend werden unterschiedliche Anwendungsbeispiele für Flüssigkeitslinsensysteme der vorstehend beschriebenen Art dargestellt.

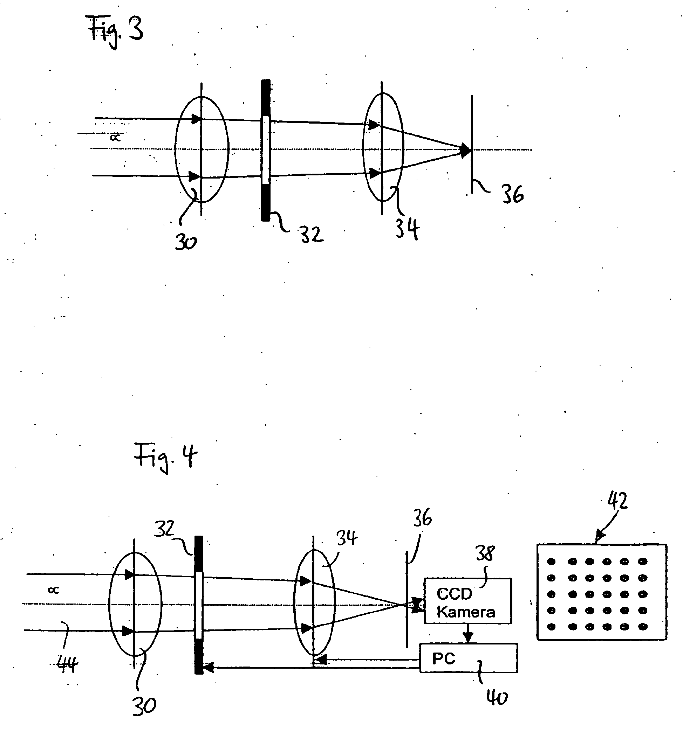

Figur 3 zeigt ein sogenanntes Kunstauge, also eine Vorrichtung mit deren Hilfe die Fehlsichtigkeit des menschlichen Auges für Untersuchungszwecke etc. nachgebildet werden kann.

In Figur 3 kommt die Strahlung von links. Eine erste Unse 30 dient dazu, den Hornhautkrümmungsradius der Hornhaut des Auges nachzubilden. Danach passiert die Strahlung eine Pupille 32, deren Blende einstellbar ist, zum Beispiel elektrisch oder mechanisch. Danach passiert die Strahlung ein Flüssigkeitslinsensystem 34 der anhand der Figuren 1 und 2 beschriebenen Art. Das Flüssigkeitslinsensystem 34 ermöglicht also, Linsenfehler elektrisch einzustellen durch wahlweise Anlegung unterschiedlicher Spannungen U1, ...., U8. Mit dem beschriebenen optischen System wird die Strahlung auf eine (künstliche) Netzhaut 36 abgebildet, wo sie untersucht werden kann.

Figur 4 zeigt eine Ausgestaltung des Kunstauge gemäß Figur 3 derart, dass reproduzierbare Messergebnisse gewonnen werden können, bei denen z.B. temperaturbedingte Änderungen der Oberflächenspannung des Flüssigkeitstropfens kompensiert sind. Auch Steuerspannungsschwankungen können damit ausgeregelt werden.

Hierzu wird gemäß Figur 4 hinter der (künstlichen) Netzhaut, die für die Strahlung teildurchlässig ist, eine Festkörperbildkamera 38 (zum Beispiel eine CCD-Kamera) angeordnet. Die mit der Kamera 38 gewonnenen elektrischen Bildsignale werden in einem Rechner 40 verarbeitet. Mit dem Rechner 40 werden in der durch Pfeile angedeuteten Weise die Pupille 32 und das Flüssigkeitslinsensystem 34 angesteuert.

Ein Bündel 44 von parallelen Lichtstrahlen, die zum Beispiel mit einem Laserstrahl und einer matrixartigen Lochblende erzeugt werden, fällt gemäß Figur 4 von links parallel in das Kunstauge ein. Zum Beispiel können hundert parallele Lichtstrahlen das Bündel 44 bilden. Das von den Lichtstrahlen im Bereich der Netzhaut 36 in der Kamera 38 erzeugte Bild ist in Figur 4 mit dem Bezugszeichen 42 dargestellt (nur 30 Strahlen, der Einfachheit halber).

Die Positionen der einzelnen Lichtstrahlen, wie sie vom Kunstauge abgebildet werden, werden mit der Kamera 38 vermessen. Aus der Lage der einzelnen Laserstrahlen kann der Lonsenfehler genau ermittelt werden. Das Messergebnis dient entweder zur Beurteilung des Linsenfehlers oder zur Ermittlung einer Unsenfehlerkorrektur, indem über die genannten Steuerspannungen U1 bis U8 Abweichungen vom erwarteten Linsenfehler korrigiert werden. Mit dem eingebauten Flüssigkeitslinsensystem 34 lässt sich die Sphäre und der Zylinder des Kunstauges elektrisch einstellen.

Das in den Figuren 3 und 4 dargestellte Ausführungsbeispiel lässt sich dahingehend erweitern, dass mehrere Flüssigkeitslinsensystem der beschriebenen Art im Strahlengang gestaffelt hintereinander angeordnet werden, wodurch sich der Messbereich und die Einstellgenauigkeit erhöhen.

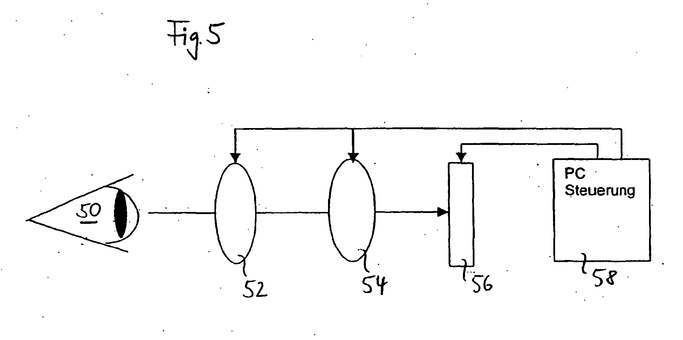

Figur 5 zeigt eine Anwendung von Flüssigkeitslinsensystemen gemäß den Figuren 1 und 2 in einem sogenannten Akkommodationsmessgerät.

Das Messgerät gemäß Figur 5 ist relativ einfach aufgebaut und ermöglicht die Messung der Akkommodationsfähigkeit eines menschlichen Auges mit hoher Genauigkeit.

Ein Gegenstand wird auf der Netzhaut des zu untersuchenden Auges 50 als Bild über zwei Flüssigkeitslinsensysteme 52, 54 abgebildet. Der Bildschirm 56 fungiert gleichzeitig als Gegenstand und als Target. Als Bildschirm 56 findet bevorzugt ein TFT-Bildschirm Verwendung, alternativ auch zum Beispiel ein CRT-Bildschirm. Bei der Messung brauchen keine mechanisch beweglichen Teile eingesetzt zu werden. Ein Rechner 58 steuert die beiden Flüssigkeitslinsensysteme 52, 54 und den Bildschirm 56. Diese Anordnung ermöglicht besonders vorteilhaft, dass mit dem Bildschirm 56 die Helligkeit und die Bildgröße so gesteuert werden, dass das abgebildete Bild unabhängig von der Fehlsichtigkeit des untersuchten Auges gleiche Helligkeit und Größe behällt. Damit ist eine genauere Messung der Akkommodationsfähigkeit möglich. Zur Messung der Akkommodationsfähigkeit kann zum Beispiel durch Anlegung geeigneter Spannungen U1, ...., U8 mittels des Rechners 58 mit dem ersten Flüssigkeitslinsensystem 52 eine Sphäre im Millisekundenbereich verändert werden. Synchron kann mit dem Flüssigkeitslinsensystem 54 der Zylinder kompensiert werden. Durch Änderung der Frequenz der erzeugten Bildänderung und Untersuchung des auf der Netzhaut abgebildeten Bildes mittels des Bildschirmes 56 kann dann die Akkommodationsfähigkeit des untersuchten Auges 50 ermittelt werden.

Durch Hinzufügung von weiteren Flüssigkeitslinsensystemen oder auch normalen Linsen zur Anordnung gemäß Figur 5 kann der Messbereich und auch die Messgenauigkeit erhöht werden.

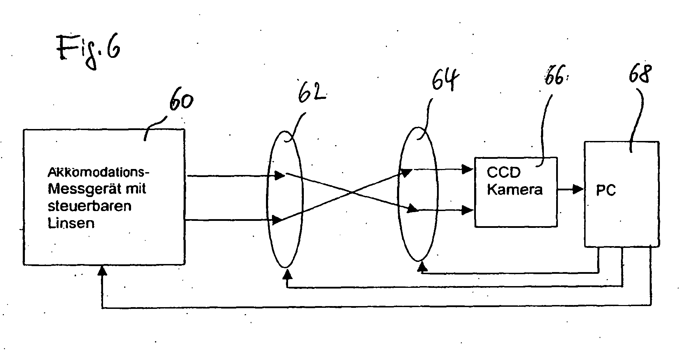

Figur 6 zeigt eine Anordnung, die als Dioptriefernrohr verwendbar ist, also ein Messgerät zur Bestimmung des Dioptriewertes eines zu untersuchenden Auges. In Figur 6 ist der Block 60 als Akkommodationsmessgerät mit steuerbaren Linsen ausgewiesen, der Block 60 kann also ein System darstellen, wie es vorstehend anhand der Figur 5 beschrieben ist. Mit der Anordnung gemäß Figur 6 kann z.B. ein Akkommodationsmessgerät 60 überprüft und kalibriert werden.

Nach Figur 6 sind im Strahlengang nach dem Akkommodationsmessgerät zwei Flüssigkeitslinsensysteme 62, 64 angeordnet. Die damit erzeugte Strahlung wird in eine CCD-Kamera 66 abgebildet. Die Bildsignale der Kamera 66 werden in einem Rechner 68 verarbeitet. Der Rechner 68 steuert in der durch Pfeile angedeuteten Weise das Akkommodationsmessgerät, das Festkörperlinsensystem 62 und das Festkörperlinsensystem 64, bei Letzteren also die oben erläuterten einstellbaren Spannungen U1, ..., U8.

Mit den genannten Flüssigkeitslinsensystemen lassen sich sowohl die Brechkraft einstellen als auch der Zylinder kompensieren. Eine Software im Rechner 68 wertet das digital gewonnene Bild aus.

Die Anordnung gemäß Figur 6 ist klein und kompakt. Sie ist ausschließlich elektrisch steuerbar und ermöglicht eine einfache Kalibrierung, womit auch eine Zeitersparnis bei der Kalibrierung verbunden ist. Auf eine aufwendige Mechanik und Optik kann also verzichtet werden. Auch ermöglicht der Aufbau gemäß Figur 6 eine einfache Dokumentierung des Kalibriervorganges mittels des Rechners und er erlaubt eine objektive Kalibrierung.

Zur Kalibrierung des Akkommodationsmessgerätes 60 wird mit dem ersten Flüssigkeitslinsensystem 62 eine definierte Brechkraft eingestellt (z.B. -3 dpt) und mittels des Flüssigkeitslinsensystems 64 wird ein definierter Zylinder eingestellt (z.B. 1 dpt 20°).

Anschließend werden die Brechkraft und der Zylinder im Akkomodationsmessgerät mit den genannten steuerbaren Linsen kompensiert. Die Kamera 66 nimmt ein Bild auf und der Rechner 68 wertet mittels Bildbearbeitungssoftware dieses Bild aus. Dabei muss die Bildgröße der Soll-Bildgröße entsprechen, ansonsten muss die Kompensation im Akkommodationsmessgerät solange fortgesetzt werden bis das mit der Kamera 66 aufgenommene Bild die erwartete Bildgröße erreicht hat. Die Steuerung der Linsen im Akkommodationsmessgerät 60 übernimmt auch der Rechner 68.

Weiterhin kann mit der Anordnung gemäß Figur 6 auch ein Akkommodationsmessgerät überprüft werden. Hierzu werden im Akkommodationsmessgerät eine definierte Brechkraft (z.B. -3 dpt) und ein definierter Zylinder (z.B. 1 dpt 20°) eingestellt. Das Dioptriefernrohr gemäß Figur 6 wird dann auf die gleiche Brechkraft (z.B. -3 dpt) und den gleichen Zylinder (z.B. 1 dpt 20°) eingestellt. Ist das Akkommodationsmessgerät richtig kalibriert, so muss die Bildgröße des mit der Kamera 66 gemessenen Bildes mit dem Soll-Bild übereinstimmen.

Auch das Dioptriefernrohr gemäß Figur 6 kann durch Kombination weiterer Flüssigkeitslinsensysteme, gegebenenfalls mit Glas- oder auch Kunststofflinsen, im Messbereich erweitert und hinsichtlich der Messgenauigkeit durch Vorkompensation verbessert werden.

## Patentansprüche

1. Kunstauge, also eine Vorrichtung mit deren Hilfe die Fehlsichtigkeit des menschlichen Auges für Untersuchungszwecke etc. nachgebildet werden kann, das in folgender Reihenfolge umfasst:
- eine Unse (30), die eine Hornhautkrümmung nachbildet;
- eine Pupille (32) mit einer Blende, die einstellbar ist;
- ein Flüssigkeitslinsensystem (34); und
- eine künstliche Netzhaut (36);
wobei das Flüssigkeitslinsensystem (34) enthält:
- einen Flüssigkeitstropfen (10); dessen Brechungselgenshaft durch elektrische Felder beeinflussbar ist;
- eine Mehrzahl von Elektroden (20a, 20b, 20c, 20d, 20e, 20f, 20g, 20h); die um den Flüssigkeitstropfen (10) herum angeordnet sind; und
- eine elektrische Spannungsversorgung, um wahlweise zur Erzeugung unterschiedlicher elektrischer Felder verschiedene Spannungen an die Elektroden anzulegen.

2. Kunstauge gemäß Anspruch 1, mit einer Laserstrahlquelle zur Erzeugung eines Bündels (44) aus einer Vielzahl paralleler Lichtstrahlen, einer CCD-Kamera (38) zur Aufnahme von von den Lichtstrahlen nach Durchgang durch die Linsen (30, 34) erzeugten Bildern (42), und mit einem Rechner (40) zur Verarbeitung der Bilder und zum Steuern des Flüssigkeitsllnsensystems in Abhängigkeit von der Bildverarbeitung.

## Claims

1. Artificial eye, i.e. a device with the aid of which the visual defect of the human eye can be replicated for examination purposes etc., which comprises in the following sequential order:
- a lens (30) replicating a corneal curvature;
- a pupil (32) comprising a diaphragm which is adjustable;
- a liquid lens system (34); and
- an artificial retina (36);
wherein the liquid lens system (34) comprises:
- a liquid drop (10) whose refractive property can be influenced by electrical fields;
- a plurality of electrodes (20a, 20b, 20c, 20d, 20e, 20f, 20g, 20h), which are arranged around the liquid drop (10); and
- an electrical voltage supply for selectively applying various voltages to the electrodes in order to generate different electrical fields.

2. Artificial eye according to claim 1, comprising a laser beam source for generating a beam (44) comprising a multiplicity of parallel light rays, a CCD camera (38) for recording images generated by the light rays after they pass through lenses (30, 34), and a computer (40) for processing the images and for controlling the liquid lens system as a function of the image processing.

## Revendications

1. OEil artificiel, soit un dispositif permettant l'émulation de l'amétropie de l'oeil aux fins d'examen, etc., comprenant dans l'ordre suivant :
- une lentille (30) qui simule la courbure de la cornée ;
- une pupille (32) avec un diaphragme ajustable ;
- un système de lentilles liquides (34) ; et
- une rétine artificielle (36) ;
ledit système de lentilles liquides (34) comprenant :
- une goutte de liquide (10) dont la caractéristique de réfraction peut être influencée par des champs électriques ;
- une pluralité d'électrodes (20a, 20b, 20c, 20d, 20e, 20f, 20g, 20h) disposées autour de la goutte de liquide (10) ; et
- une alimentation en tension électrique pour mettre, au choix, les électrodes sous tensions différentes afin d'engendrer différents champs électriques.

2. OEil artificiel selon la revendication 1, avec une source de rayon laser pour engendrer un faisceau (44) constitué par un grand nombre de rayons lumineux parallèles, une caméra CCD (38) pour enregistrer les images (42) générées par les rayons lumineux après qu'ils ont traversé les lentilles (30, 34), et un ordinateur (40) pour le traitement des images et la commande du système de lentilles liquides en fonction du traitement des images.
